# FASCICULE DE BREVET EUROPEEN

(11) **EP 3 160 539 B1**
(45) Date de publication et mention de la délivrance du brevet: **04.04.2018**
(21) Numéro de dépôt: 15753077.5
(22) Date de dépôt: 19.06.2015
(51) Int. Cl.: A61M 1/36, B01D 39/16, A61M 1/02

(54) **UNITÉ DE FILTRATION POUR LA DÉLEUCOCYTATION DU SANG COMPRENANT UN PRÉFILTRE**
FILTRIERUNGSEINHEIT ZUR LEUKOREDUKTION VON BLUT MIT EINEM VORFILTER
FILTERING UNIT FOR LEUKOREDUCTION OF THE BLOOD COMPRISING A PREFILTER

(30) Priorité: 26.06.2014 FR 1456012
(43) Date de publication de la demande: 03.05.2017
(73) Titulaire: Maco Pharma, 59400 Mouvaux (FR)
(72) Inventeur: DUCOROY, Laurent, 59800 Lille (FR); BAUDOIN, Matthieu, 08000 Warq la Mal Campée (FR)
(74) Mandataire: Sayettat, Julien Christian
(86) Numéro de dépôt international: PCT/FR2015/051634
(87) Numéro de publication internationale: WO 2015/197955

(56) Documents cités:
- EP-A1- 2 818 223
- FR-A1- 2 250 540
- FR-A1- 2 892 949

## Description

L'invention concerne une unité de filtration destinée à éliminer les leucocytes d'un composant sanguin ainsi qu'un système à poches comprenant une telle unité.

L'invention s'applique typiquement à la filtration du sang ou d'un composant sanguin, et plus particulièrement à l'élimination des leucocytes d'un sang total ou d'un concentré de globules rouges.

Le sang ou un composant sanguin, après son recueil et sa séparation dans le cas d'un composant, est notamment destiné à être transfusé à un patient qui en a besoin. Lors de cette transfusion, il est bien connu que les leucocytes sont indésirables en ce qu'ils sont susceptibles de provoquer chez le patient des réactions gênantes et/ou potentiellement dangereuses. En effet, les leucocytes augmentent le risque de rejet immunitaire tel que la maladie du greffon contre l'hôte et favorisent la transmission d'agents infectieux.

C'est pourquoi il est recommandé, voire imposé dans certains pays, de déleucocyter le sang ou le composant sanguin préalablement à sa transfusion, et ce dans un rendement donné.

A ce jour, la solution optimale pour éliminer les leucocytes est de filtrer le sang ou le composant sanguin au travers d'une unité de filtration pourvue d'un milieu de déleucocytation.

Un tel milieu de déleucocytation comprend une ou plusieurs membrane(s) et/ou une ou plusieurs couche(s) de non-tissé réalisée(s) en un matériau polymère et choisie(s) de façon à améliorer le taux de déleucocytation, la récupération des composants sanguins, le temps de filtration et/ou la sélectivité de la filtration.

Les non-tissés sont des matériaux constitués d'un enchevêtrement de fibres, à l'exclusion des matériaux de fibres tissées ou tricotées.

Pour réaliser un non-tissé, il existe deux grands types de matière première : d'une part les fibres discontinues conditionnées dans des "balles" et d'autre part des fibres continues (ou filaments) obtenues par extrusion de polymère(s).

Pour former un non-tissé à partir de fibres discontinues, on utilise la voie humide qui est similaire au procédé papetier ou la voie sèche. Dans la voie sèche, les fibres discontinues sont soit travaillées à l'aide d'une carde pour former un voile, soit dispersées dans un flux d'air selon un procédé aérodynamique. Après le cardage, les fibres sont généralement orientées dans le sens de défilement de la machine.

La technique de formation du non-tissé par voie sèche fournit une structure ayant une faible cohésion. La consolidation s'effectue de façon chimique (agent liant de type acrylique), mécanique (aiguilletage ou enchevêtrement par jet d'eau) ou thermique.

La plupart des milieux de déleucocytation comprennent, quant à eux, des non-tissés formés à partir de filaments. Ces non-tissés sont réalisés par voie fondue ou par filage direct. Le non-tissé filé lié (spun-bond) permet d'obtenir des fibres ayant un diamètre généralement inférieur à 20 µm. Dans le cas de fibres soufflées à l'état fondu (melt-blown) obtenues par une technique d'extrusion soufflage, les fibres ont généralement un diamètre inférieur à 5 µm. Les non-tissés de type spun-bond ou melt-blown possèdent donc une structure relativement dense apte à retenir les leucocytes par un mécanisme d'adsorption et/ou de filtration en surface (ou tamisage).

En plus d'un milieu de déleucocytation, les unités de filtration comprennent également généralement un préfiltre destiné à éliminer les micro-agrégats. Par exemple, dans le document EP 1 336 417, une couche de polyester ayant une perméabilité comprise entre 1000 et 5000 L/m²/s et une taille de pores d'environ 35 µm est disposée en amont du milieu de déleucocytation.

Selon les pays et les habitudes des banques de sang, les composants sanguins peuvent être stockés avant filtration, jusqu'à 14 jours, à 4°C ou à température ambiante, avec ou sans solution additive comme par exemple la solution saline adénine glucose mannitol (SAGM).

Dans ces conditions, les temps de filtration avec des unités de filtration du type de celles décrites dans le document EP 1 336 417 peuvent augmenter jusqu'à une durée pouvant dépasser une heure. Il arrive également que ces unités de filtration colmatent, de sorte que la filtration s'arrête et le composant sanguin est perdu.

Ces colmatages et temps de filtration allongés sont dus essentiellement à la présence d'agrégats formés notamment de globules rouges, de plaquettes, de gel de fibrine. La taille de ces agrégats augmente avec la durée de stockage, pouvant aller de 20 µm jusqu'à 200 µm.

Afin d'éliminer ces agrégats, le document US 4,923,620 propose une unité de filtration comprenant trois éléments : un voile aiguilleté avec un diamètre de fibre compris entre 20 et 30 µm pour éliminer les gels et agrégats, deux couches intermédiaires ou plus de fibres non-tissées de type melt-blown pour éliminer les micro-agrégats, et plusieurs couches finales de fibres non-tissées de type melt-blown pour éliminer les leucocytes, ces fibres ayant un diamètre plus petit que celui des fibres des couches intermédiaires. Le voile aiguilleté comprend un liant de type acrylique et est compressé à chaud afin de réduire sa taille de pores jusqu'à environ 50 µm.

Le document EP 2 286 821 décrit un matériau de filtre pour éliminer les agrégats comprenant d'une part des fibres courtes ayant un titrage compris entre 0,7 et 4 décitex (dtex) et une longueur comprise entre 1 et 80 mm et d'autre part un tissu de fond comprenant des fibres longues de type spun-bond. Les fibres courtes ont une structure tridimensionnelle et sont enchevêtrées, notamment par jet d'eau, dans les fibres longues, de sorte à obtenir une masse surfacique comprise entre 10 et 80 g/m².

Dans ces deux documents, le matériau pour éliminer les agrégats est consolidé de façon mécanique (aiguilletage ou hydro-enchevêtrement), ce qui peut engendrer des zones d'écoulement préférentiel qui nuisent à l'efficacité de la fiiltration.

Enfin, dans le document WO2013/110694, il est proposé d'utiliser des fibres présentant une cannelure, notamment des fibres trilobées de type spunbond ou melt-blown, afin d'éliminer des produits sanguins les agrégats, les gels, les débris cellulaires et autres fragments. Le document FR 2 892 949 prévoit une unité de filtration destinée à permettre la déleucocytation du sang ou d'un composant sanguin avec un préfiltre pour éliminer les agrégats.

L'invention propose une unité de filtration pourvue d'un préfiltre permettant de réduire de façon significative les risques de colmatage aussi bien au niveau du préfiltre que du milieu de déleucocytation, et cela, sans créer de passages préférentiels dans le milieu de déleucocytation notamment. L'invention propose également un préfiltre ayant une structure suffisamment perméable pour retenir les agrégats sans risque de colmatage tout en ayant une tenue mécanique suffisante pour être intégrée dans une unité de filtration.

A cet effet et selon un premier aspect, l'invention concerne une unité de filtration destinée à permettre la déleucocytation du sang ou d'un composant sanguin, comprenant une enveloppe extérieure munie d'au moins un orifice d'entrée et d'au moins un orifice de sortie, l'enveloppe renfermant un élément poreux interposé entre lesdits orifices, ledit élément poreux renfermant au moins un préfiltre pour éliminer les agrégats et au moins un milieu de déleucocytation par adsorption et/ou par filtration des leucocytes, le préfiltre pour éliminer les agrégats comprenant au moins un voile non-tissé cohérent comprenant un mélange de premières fibres thermoplastiques et discontinues et de deuxièmes fibres thermoplastiques et discontinues dont un point de fusion est inférieur à celui desdites premières fibres, lesdites premières et deuxièmes fibres étant liées par des jonctions thermiques les unes aux autres.

Selon un autre aspect, l'invention concerne un système à poches pour la déleucocytation d'un fluide tel que le sang ou un composant sanguin comprenant une poche de recueil du filtrat, ladite poche étant reliée, par l'intermédiaire d'une tubulure et au niveau d'un orifice d'entrée, à un orifice de sortie d'une unité de filtration selon le premier aspect de l'invention.

D'autres objets et avantages apparaîtront au cours de la description qui suit.
La figure 1 représente de façon schématique une vue en coupe d'une unité de filtration selon l'invention.
La figure 2 représente une image au microscope électronique à balayage d'un voile de préfiltre utilisé dans une unité de filtration de l'invention.
La figure 3 représente de façon schématique un système à poches comprenant une unité de filtration selon l'invention.

L'unité de filtration selon l'invention est destinée à permettre la déleucocytation du sang ou d'un composant sanguin.

Par composant sanguin, on entend notamment les concentrés de globules rouges, les concentrés plaquettaires, le plasma, le plasma pauvre ou riche en plaquettes, et la couche leuco-plaquettaire ou buffy coat. Les concentrés de globules rouges sont obtenus par centrifugation douce d'une unité de sang total puis extraction de la couche de plasma riche en plaquettes. Dans une variante, les concentrés de globules rouges sont obtenus par centrifugation dure d'une unité de sang total puis extraction de la couche de plasma pauvre en plaquette. Les concentrés de globules rouges sont alors dits "non appauvris en leucocytes et en plaquettes". Dans une autre variante, les concentrés de globules rouges sont obtenus par centrifugation dure d'une unité de sang total puis extraction de la couche de buffy coat et de la couche de plasma pauvre en plaquettes. Les concentrés de globules rouges sont alors dits "appauvris en leucocytes et en plaquettes".

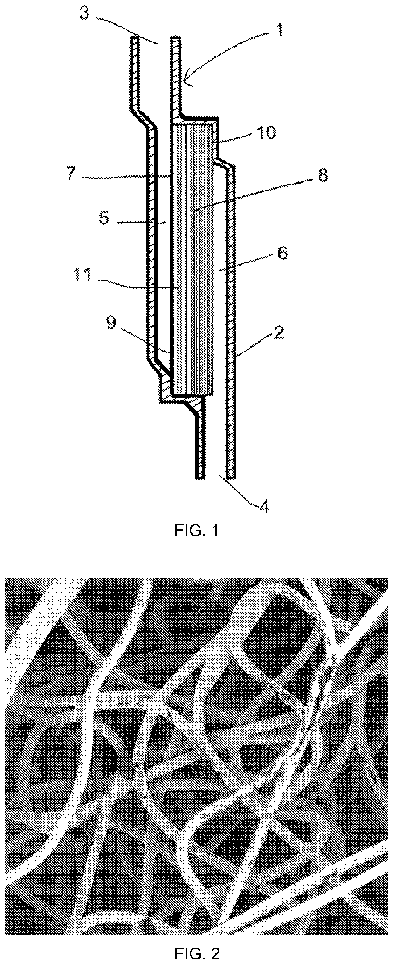

En relation avec la figure 1, l'unité de filtration 1 comprend une enveloppe extérieure 2 munie d'au moins un orifice d'entrée 3 et d'au moins un orifice de sortie 4, l'enveloppe 2 renfermant un élément poreux interposé entre lesdits orifices 3,4. L'élément poreux forme avec l'enveloppe 2 un compartiment d'entrée 5 destiné à recevoir le fluide à filtrer, ledit compartiment d'entrée 5 étant en communication avec l'orifice d'entrée 3, et un compartiment de sortie 6 destiné à recueillir le filtrat, ledit compartiment de sortie 6 étant en communication avec l'orifice de sortie 4.

L'enveloppe extérieure 2 de l'unité de filtration est souple, rigide ou semi-rigide. Par exemple, l'enveloppe est réalisée en polycarbonate, en polychlorure de vinyle ou en une polyoléfine, telle que le polypropylène, le polyéthylène ou un complexe à base de polypropylène.

Le sens d'écoulement du fluide dans l'unité de filtration, de l'entrée 3 vers la sortie 4, permet de définir les termes "amont" et "aval" utilisés dans la description.

L'élément poreux renferme au moins un préfiltre 7 pour éliminer les agrégats et au moins un milieu de déleucocytation 8 par adsorption et/ou par filtration des leucocytes. Le préfiltre 7 est disposé en amont du milieu de déleucocytation 8.

Selon l'invention, le préfiltre 7 pour éliminer les agrégats comprend au moins un voile 9 non-tissé cohérent comprenant un mélange de premières fibres thermoplastiques discontinues et de deuxièmes fibres thermoplastiques discontinues dont un point de fusion est inférieur à celui desdites premières fibres, lesdites premières et deuxièmes fibres étant liées par des jonctions thermiques les unes aux autres.

Le voile 9 du préfiltre est de type non-tissé, c'est-à-dire que les fibres le constituant sont enchevêtrées entre elles, mais ne sont ni tissées ni tricotées.

Le voile 9 du prefiltre est cohérent, c'est-à-dire que les fibres le constituant possède une résistance à la séparation. Par exemple, les fibres montrent une cohésion en adhérant étroitement entre elles et en résistant à la déchirure et à l'étirement.

Après de nombreux essais, la demanderesse a identifié que les matériaux non-tissés formés à partir de fibres discontinues constituaient un matériau de préfiltre particulièrement adapté pour retenir les agrégats et autres gels potentiellement présents dans le sang ou le composant sanguin à filtrer.

En particulier, la longueur de ces fibres discontinues est comprise dans la plage allant de 25 mm à 120 mm.

Le titrage de ces fibres, qui correspond à la masse en gramme d'une longueur de 10 000 mètres de fil est notamment compris dans la plage allant de 0,5 à 15 dtex.

Les fibres présentes dans ledit voile 9 du préfiltre sont des fibres thermoplastiques d'un polymère biocompatible tel que le polyester, le polypropylène, le polyéthylène, le polyamide, la cellulose, ou les mélanges de ces polymères. En particulier, les fibres sont des fibres de polyester, notamment de polyéthylène téréphtalate.

En variante, les fibres sont des fibres bi-composantes ou multi-composantes, réalisées à partir de deux composants polymères distincts ou plus, co-extrudés pour former une seule fibre. En particulier, les fibres bi-composantes sont de type segmentées ou îles-en-mer (ou islands-in-the-sea) selon la structure de leur section. Les fibres segmentées sont des fibres dont les composants sont situés côte à côte. Les fibres îles-en-mer sont des fibres dont l'un des composants (l'âme) est entouré par l'autre composant (la peau). Plus particulièrement, l'un des composants possède un point de fusion inférieur à l'autre.

Le matériau du voile 9 du préfiltre non-tissé utilisé dans l'unité de l'invention est formé par voie sèche, notamment par cardage, ce qui permet d'obtenir une structure plus aérée que les structures obtenues à partir de filaments, et donc plus susceptible d'arrêter les agrégats de taille relativement importante sans bloquer le flux de sang.

Selon une réalisation avantageuse, le voile du préfiltre est un voile cardé thermolié pour le rendre cohérent.

La demanderesse a identifié que la consolidation thermique du voile cardé, et notamment la consolidation par traversée d'air permettait d'obtenir un voile suffisamment perméable pour retenir les agrégats sans risque de blocage et ayant une résistance mécanique suffisante pour être intégré dans une unité de déleucocytation. En effet, le voile doit pouvoir subir des étapes de déroulage, découpe et soudure sans modifier ses propriétés physiques.

En outre, la consolidation thermique par traversée d'air offre l'avantage supplémentaire par rapport à une consolidation thermique par calandrage de ne pas modifier l'état de surface du voile et de former une structure plus aérée facilitant l'amorçage de la filtration.

Contrairement à la consolidation mécanique, la technique de consolidation thermique évite la formation de passages préférentiels dans le voile. D'autre part, la consolidation thermique ne nécessite pas l'utilisation de substances chimiques susceptibles d'interagir avec les composants du sang.

La consolidation thermique par traversée d'air assure ainsi une stabilité dimensionnelle, notamment lors de la stérilisation par la chaleur humide de l'unité de filtration.

Notamment, les fibres du voile 9 du préfiltre sont liées par un procédé de liaison par traversée d'air.

De façon avantageuse pour réaliser la consolidation thermique par traversée d'air, les deuxièmes fibres thermoplastiques sont des fibres bi-composantes réalisées à partir de deux polymères distincts co-extrudés pour former une seule fibre. Ces fibres bi-composantes sont "segmentées" ou "îles-en-mer".

En particulier, les deuxièmes fibres thermoplastiques sont des fibres discontinues courtes, de longueur comprise dans la plage allant de 25 et 60 mm.

Avantageusement, les deuxièmes fibres sont des fibres de type îles-en-mer comprenant une âme en un premier polymère thermoplastique enveloppé par une peau d'un deuxième polymère thermoplastique ayant un point de fusion inférieur à celui du premier polymère et inférieur à celui du polymère constituant les premières fibres.

Par passage d'air chaud à une température déterminée, la peau des deuxièmes fibres va fondre, créant ainsi des jonctions thermiques avec les autres fibres du voile.

La figure 2 montre une image prise au microscope électronique à balayage d'un voile cardé thermolié par traversée d'air. Sur cette image, on voit les fibres bi-composantes qui ont partiellement fondues pour former des jonctions thermiques avec les autres fibres du voile.

De façon avantageuse, les premières et deuxièmes fibres sont réalisées dans une même famille de polymère. Par exemple, les premières fibres sont réalisées en polyéthylène téréphtalate (PET) et les deuxièmes fibres sont des fibres bi-composantes comprenant une âme en polyéthylène téréphtalate (PET) recouverte d'un copolymère de polyéthylène téréphtalate (Co-PET). L'âme des deuxièmes fibres est ainsi réalisée dans le même matériau que les premières fibres.

Selon une réalisation, le titrage des premières fibres est supérieur ou égal à celui des deuxièmes fibres. Par exemple, le titrage des premières fibres est compris dans la plage allant de 5 à 15 dtex et celui des deuxièmes fibres est compris dans la plage allant de 0,5 et 5 dtex.

La proportion des fibres bi-composantes dans le matériau de préfiltre dépend des matériaux polymères utilisés. En particulier, le voile 9 du préfiltre comprend entre 50 et 80% en masse de fibres bi-composantes. Les fibres bi-composantes sont alors réparties de façon relativement homogène dans le voile 9, de façon à créer des jonctions thermiques en nombre suffisant et réparties dans tout le volume du voile afin d'assurer sa cohésion.

Avantageusement, le voile 9 non-tissé du préfiltre comprend des troisièmes fibres thermoplastiques discontinues. Ces troisièmes fibres sont d'un type différent de celui des premières et deuxièmes fibres, c'est-à-dire qu'elles diffèrent par leur composition et/ou leur propriété physique.

La présence de ces troisièmes fibres dans le matériau constituant le voile 9 du préfiltre permet d'obtenir une grande mixité de fibres qui améliore la cohésion du voile.

Dans une réalisation particulière, le titrage des troisièmes fibres est compris dans la plage allant de 0,5 à 5 dtex. De façon additionnelle ou alternative, les premières et troisièmes fibres sont réalisées dans un même polymère thermoplastique. Par exemple, les premières et troisièmes fibres sont des fibres de polyester tel que le polyéthylène téréphtalate.

Dans ce cas et selon un exemple particulier, la longueur des premières fibres est différente de celle des troisièmes fibres.

Dans le cas où le voile 9 du préfiltre contient trois types de fibres, le voile 9 du préfiltre comprend entre 50 et 80% en masse de fibres bi-composantes.

Bien que le voile 9 du préfiltre ne retienne pas ou peu de leucocytes, les essais ont montré que les caractéristiques physiques du voile 9 du préfiltre influent sur l'efficacité de la rétention de leucocytes du milieu de déleucocytation.

Selon une réalisation, le voile 9 du préfiltre présente une perméabilité à l'air comprise dans la plage allant de 3 000 à 12 000 L/m²/s.

La perméabilité à l'air est déterminée selon la norme NF EN ISO 9237 sur un échantillon d'au moins 100 cm² à l'aide d'un perméabilimètre à l'air tel que le FX 3300 de TextTest avec une pression d'air réglée sur 196 Pa (Norme EDANA 140.1).

Lorsque la perméabilité à l'air du voile 9 du préfiltre est supérieure à 12 000 L/m²/s, des essais ont montré que le temps de filtration diminuait, puisque le sang a potentiellement plus de place pour circuler. Cependant, le nombre de leucocytes résiduels augmente aussi, du fait des passages préférentiels créés dans le milieu de déleucocytation par les agrégats non retenus par le préfiltre et qui vont colmater à certains endroits le milieu de déleucocytation. En dessous de 3 000 L/m²/s, le temps de filtration augmente ainsi que le risque de blocage.

Une perméabilité à l'air comprise dans la plage allant de 3 500 à 5 500 L/m²/s est un bon compromis entre le temps de filtration, l'occurrence des blocages et le taux de déleucocytation.

Selon une réalisation, le voile 9 du préfiltre présente une masse surfacique comprise dans la plage allant de 40 à 90 g/m², notamment compris dans la plage allant de 50 à 80 g/m².

En dessous de 50 g/m², il apparaît que le taux de déleucocytation se dégrade considérablement, du fait de la densité trop faible de fibres. Au dessus de 90 g/m², la densité de fibre est trop grande et le risque de blocage augmente.

Pour une masse surfacique comprise entre 40 et 90 g/m², l'épaisseur du voile 9 du préfiltre est comprise entre 300 et 1 000 µm, mesurée à l'aide d'un micromètre avec une pression de 10 kPa (norme ISO 9073-2:1995). Une épaisseur comprise entre 600 et 850 µm est avantageuse pour ne pas trop augmenter l'épaisseur totale de l'élément poreux et, par voie de conséquence, la compression de l'élément poreux dans l'enveloppe de l'unité de filtration.

Le voile 9 du préfiltre présente une taille moyenne de pores comprise dans la plage allant de 60 à 300 µm, plus particulièrement d'environ 100 µm.

La taille de pores est mesurée à l'aide d'un poromètre selon la norme ISO 13485:2004.

Outre le préfiltre 7, l'élément poreux de l'unité de filtration comprend un milieu de déleucocytation 8 par adsorption et/ou par filtration des leucocytes. Ce milieu de déleucocytation 8 comprend en particulier une ou plusieurs couches 10 d'un matériau non-tissé de fibres soufflées à l'état fondu.

Par exemple, les fibres du milieu de déleucocytation 8 sont choisies parmi les fibres de polyéthylène, polypropylène, polyéthylène téréphtalate, polybutylène téréphtalate et leurs copolymères,

Chaque couche 10 du milieu de déleucocytation présente une perméabilité à l'air inférieure à celle du voile 9 du préfiltre, de sorte à créer un gradient de perméabilité décroissant de l'amont vers l'aval.

Afin de retenir au mieux les leucocytes, chaque couche 10 du milieu de déleucocytation 8 présente une perméabilité comprise dans la plage allant de 90 à 500 L/m²/s.

La masse surfacique de chaque couche 10 du milieu de déleucocytation 8 est comprise dans la plage allant de 20 à 80 g/m², notamment la plage allant de 30 à 60 g/m² pour une épaisseur comprise entre 100 et 400 µm.

Selon une variante de réalisation, le préfiltre 7 de l'unité de filtration 1 comprend en outre en aval dudit voile 9 du préfiltre, une ou plusieurs couches 11 d'un matériau non-tissé poreux choisi dans le groupe comprenant un non-tissé de fibres soufflées à l'état fondu ou un non-tissé filé lié.

Ces non-tissés du préfiltre formés à partir de filaments comprennent des fibres de polyéthylène, polypropylène, polyéthylène téréphtalate, polybutylène téréphtalate et leurs copolymères.

Ces non-tissés 11 du préfiltre formés de filaments présentent une perméabilité à l'air inférieure à celle du voile 9 du préfiltre, mais supérieure à celle de la ou les couches 10 du milieu de déleucocytation 8, de sorte à créer un gradient de perméabilité décroissant de l'amont vers l'aval dans l'élément poreux.

Par exemple, les couches 11 de non-tissés du préfiltre formés de filaments présentent une perméabilité comprise dans la plage allant de 600 à 2000 L/m²/s.

Avantageusement, le préfiltre 7 comprend un seul voile 9 cardé thermolié. En effet, un seul voile est suffisant pour limiter les risques de blocage.

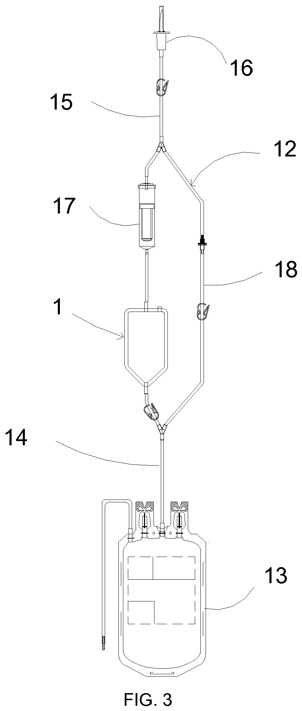

Selon un autre aspect et en relation avec la figure 3, l'invention concerne un système à poches 12 pour la déleucocytation d'un fluide tel que le sang ou un composant sanguin, comprenant une poche 13 de recueil du filtrat, ladite poche 13 étant reliée, par l'intermédiaire d'une tubulure 14 et au niveau d'un orifice d'entrée, à un orifice de sortie d'une unité de filtration 1 selon le premier aspect de l'invention.

Le système 12 comprend en outre des moyens de connexion avec une poche (non représentée) contenant le fluide à filtrer qui sont connectés, par l'intermédiaire d'une tubulure 15, à un orifice d'entrée de l'unité de filtration. Les moyens de connexion sont par exemple un perforateur 16.

Ainsi, le fluide, une fois collecté, peut être introduit dans le système à poches 12 pour être filtré au moyen de l'unité de filtration, le filtrat étant ensuite recueilli dans la poche de recueil du filtrat 13.

Une chambre compte-gouttes 17 est connectée au système sur la tubulure 15 reliant l'unité de filtration 1 et les moyens de connexion à une poche contenant le fluide à filtrer.

Une tubulure de dérivation 18 est connectée d'une part à la tubulure 14 reliant l'unité de filtration 1 et la poche 13 de recueil du filtrat, et d'autre part à la tubulure 15 reliant l'unité de filtration et les moyens de connexion à une poche contenant le fluide à filtrer, en amont de la chambre compte-gouttes 17, le cas échéant.

Cette tubulure de dérivation 18 est utilisée pour chasser l'air de la poche 13 de recueil du filtrat et pour purger l'unité de filtration 1.

D'autres systèmes à poches bien connus, comme ceux décrits dans le document EP 1 336 417 peuvent être utilisés dans le cadre de l'invention.

### Exemples

### Exemple 1 : Unité de filtration du sang total

Une unité de filtration a été réalisée comprenant, dans un boîtier rigide, un élément poreux constitué d'amont en aval et empilées l'une sur l'autre :
- un voile cardé consolidé par traversée d'air comprenant un mélange de fibres de PET et de fibres Co-PET ayant une perméabilité comprise entre 4000 et 5000 L/m²/s, une masse surfacique comprise entre 50 et 70 g/m², et une taille moyenne de pores d'environ 100 µm,
- entre une et trois couches de non-tissé en polybutylène téréphthalate melt-blown ayant chacune une masse surfacique d'environ 40 g/m², une épaisseur d'environ 320 µm, et une perméabilité à l'air d'environ 1000 L/m²/s, en tant que couches de préfiltre.
- entre vingt-cinq et trente-cinq couches de non-tissé en polybutylène téréphthalate melt-blown ayant chacune une masse surfacique d'environ 52 g/m² et une perméabilité à l'air d'environ 130 L/m²/s, en tant que milieu de déleucocytation.

Cette unité de filtration est utilisée pour éliminer les agrégats et les leucocytes d'un sang total humain (450-480 mL) additionné d'un anticoagulant (CPD) et conservé jusqu'à 24 h après le prélèvement à température ambiante.

### Exemple 2 : Unité de filtration d'un concentré de globules rouges appauvri en leucocytes et en plaquettes

Un filtre souple de la gamme Leucoflex destiné à la filtration en ligne des concentrés de globules rouges et commercialisé par Maco Pharma a été modifié en remplaçant une couche de préfiltre par un voile cardé consolidé par traversée d'air comprenant un mélange de fibres de PET et de fibres Co-PET ayant une perméabilité comprise entre 4000 et 5000 L/m²/s, une masse surfacique comprise entre 50 et 70 g/m², et une taille moyenne de pores d'environ 100 µm.

Cette unité de filtration est utilisée pour éliminer les agrégats et les leucocytes d'un concentré de globules rouges qui a été préparé à partir d'un sang total (450-480 mL), et qui a été conservé jusqu'à 24 h à température ambiante.

### Exemple 3 : Filtration d'un concentré de globules rouges

Une unité de filtration a été réalisée comprenant, dans un boîtier rigide, un élément poreux constitué d'amont en aval et empilées l'une sur l'autre :
- un voile cardé consolidé par traversée d'air comprenant un mélange de fibres de PET et de fibres Co-PET ayant une perméabilité comprise entre 4000 et 5000 L/m²/s, une masse surfacique comprise entre 50 et 70 g/m², et une taille moyenne de pores d'environ 100 µm ;
- entre une et trois couches de non-tissé melt-blown en polypropylène ayant une épaisseur de l'ordre de 285 µm, et une perméabilité à l'air d'environ 800 l/m²/s, en tant que couches de préfiltre ;
- entre dix et vingt couches de non-tissé en polypropylène melt-blown ayant chacune une masse surfacique d'environ 40 g/m² et une perméabilité à l'air de 110 l/m²/s, en tant que milieu de déleucocytation.

Cette unité de filtration est utilisée pour éliminer les agrégats et les leucocytes d'un concentrés de globules rouges obtenu par centrifugation douce d'un prélèvement de sang total (450-480 mL) additionné d'un anticoagulant, et qui a été conservé entre 3 et 14 jours à 4°C, additionné d'une solution additive SAGM (dans ce cas l'anticoagulant est le CPD) ou non (dans ce cas, l'anticoagulant est le CPDA-1).

## Revendications

1. Unité de filtration (1) destinée à permettre la déleucocytation du sang ou d'un composant sanguin, comprenant une enveloppe extérieure (2) munie d'au moins un orifice d'entrée (3) et d'au moins un orifice de sortie (4), l'enveloppe renfermant un élément poreux interposé entre lesdits orifices, ledit élément poreux renfermant au moins un préfiltre (7) pour éliminer les agrégats et au moins un milieu de déleucocytation (8) par adsorption et/ou par filtration des leucocytes, le préfiltre (7) pour éliminer les agrégats comprenant au moins un voile (9) non-tissé cohérent **caractérisé en ce que** le voile (9) comprend un mélange de premières fibres thermoplastiques discontinues et de deuxièmes fibres thermoplastiques discontinues dont un point de fusion est inférieur à celui desdites premières fibres, lesdites premières et deuxièmes fibres étant liées par des jonctions thermiques les unes aux autres.

2. Unité de filtration selon la revendication 1, dans laquelle voile non-tissé (9) du préfiltre comprend des troisièmes fibres thermoplastiques discontinues.

3. Unité de filtration selon la revendication 2, dans laquelle les premières et troisièmes fibres sont réalisées dans un même polymère thermoplastique.

4. Unité de filtration selon l'une des revendications 1 à 3, dans laquelle les deuxièmes fibres thermoplastiques sont des fibres bi-composantes réalisées à partir de deux polymères thermoplastiques distincts co-extrudés pour former une seule fibre.

5. Unité de filtration selon la revendication 4, dans laquelle le voile (9) du préfiltre comprend entre 50 et 80% en masse de fibres bi-composantes.

6. Unité de filtration selon l'une des revendications 1 à 5, dans laquelle le titrage des premières fibres est compris dans la plage allant de 5 à 10 dtex.

7. Unité de filtration selon l'une des revendications 1 à 6, dans laquelle le titrage des deuxièmes fibres est compris dans la plage allant de 0,5 et 5 dtex.

8. Unité de filtration selon l'une des revendications 2 à 7, dans laquelle le titrage des troisièmes fibres est compris dans la plage allant de 0,5 à 5 dtex.

9. Unité de filtration selon la revendication 1 à 8, dans laquelle la longueur des fibres discontinues est comprise dans la plage allant de 25 mm à 120 mm.

10. Unité de filtration selon l'une des revendications 2 à 9, dans laquelle la longueur des premières fibres est différente de celle des troisièmes fibres.

11. Unité de filtration selon l'une quelconque des revendications 1 à 10, dans laquelle le voile (9) du préfiltre présente une perméabilité à l'air comprise dans la plage allant de 3000 à 12 000 L/m²/s.

12. Unité de filtration selon l'une des revendications 1 à 11, dans laquelle le voile (9) du préfiltre présente une masse surfacique comprise dans la plage allant de 40 à 90 g/m².

13. Unité de filtration selon la revendication 12, dans laquelle le voile (9) du préfiltre présente une masse surfacique comprise dans la plage allant de 50 à 80 g/m².

14. Unité de filtration selon l'une des revendications 1 à 13, dans laquelle le voile (9) du préfiltre est un voile cardé thermolié pour le rendre cohérent.

15. Unité de filtration selon l'une des revendications 1 à 14, dans laquelle les fibres du voile (9) du préfiltre sont liées par un procédé de liaison par traversée d'air.

16. Unité de filtration selon l'une des revendications 1 à 15, dans laquelle le milieu de déleucocytation (8) comprend une ou plusieurs couches (10) d'un matériau non-tissé de fibres soufflées à l'état fondu.

17. Unité de filtration selon la revendication 16, dans laquelle les fibres du milieu de déleucocytation (8) sont choisies parmi les fibres de polyéthylène, polypropylène, polyéthylène téréphtalate, polybutylène téréphtalate et leurs copolymères.

18. Unité de filtration selon la revendications 16 ou 17, dans laquelle chaque couche (10) du milieu de déleucocytation présente une perméabilité à l'air inférieure à celle du voile (9) du préfiltre.

19. Unité de filtration selon la revendication 18, dans laquelle chaque couche (10) du milieu de déleucocytation présente une perméabilité comprise dans la plage allant de 90 à 500 L/m²/s.

20. Unité de filtration selon l'une quelconque des revendications 1 à 19, dans laquelle le préfiltre (7) comprend en outre en aval du voile (9) du préfiltre, une ou plusieurs couches (11) d'un matériau non-tissé poreux choisi dans le groupe comprenant un non-tissé de fibres soufflées à l'état fondu ou un non-tissé filé lié.

21. Système à poches (12) pour la déleucocytation d'un fluide tel que le sang ou un composant sanguin, **caractérisé en ce qu'**il comprend une poche (13) de recueil du filtrat, ladite poche (13) étant reliée, par l'intermédiaire d'une tubulure (14) et au niveau d'un orifice d'entrée, à un orifice de sortie d'une unité de filtration (1) selon l'une des revendications 1 à 20.

## Patentansprüche

1. Filtereinheit (1), die ausgelegt ist, um die Entfernung von Leukozyten aus dem Blut oder einer Blutverbindung zu ermöglichen, umfassend eine äußere Hülle (2), die mit mindestens einer Eintrittsöffnung (3) und mit mindestens einer Austrittsöffnung (4) ausgestattet ist, wobei die Hülle ein poröses Element einschließt, das zwischen den Öffnungen angeordnet ist, wobei das poröse Element mindestens einen Vorfilter (7) einschließt, um die Aggregate und mindestens ein Medium zum Entfernen von Leukozyten (8) durch Adsorption und/oder durch Filtern der Leukozyten zu beseitigen, wobei der Vorfilter (7) zum Beseitigen der Aggregate mindestens ein kohärentes Vliestuch (9) umfasst, **dadurch gekennzeichnet, dass** das Tuch (9) eine Mischung aus ersten diskontinuierlichen thermoplastischen Fasern und zweiten diskontinuierlichen thermoplastischen Fasern umfasst, wobei ein Fusionspunkt niedriger als derjenige der ersten Fasern ist, wobei die ersten und zweiten Fasern durch thermische Anschlüsse miteinander verbunden sind.

2. Filtereinheit nach Anspruch 1, wobei das Vliestuch (9) des Vorfilters dritte diskontinuierliche thermoplastische Fasern umfasst.

3. Filtereinheit nach Anspruch 2, wobei die ersten und dritten Fasern aus einem gleichen thermoplastischen Polymer hergestellt sind.

4. Filtereinheit nach einem der Ansprüche 1 bis 3, wobei die zweiten thermoplastischen Fasern Fasern aus zwei Komponenten sind, die ausgehend von zwei verschiedenen koextrudierten thermoplastischen Copolymeren hergestellt sind, um eine einzige Faser zu bilden.

5. Filtereinheit nach Anspruch 4, wobei das Tuch (9) des Vorfilters zwischen 50 und 80 Massen-.% Fasern aus zwei Komponenten umfasst.

6. Filtereinheit nach einem der Ansprüche 1 bis 5, wobei die Titration der ersten Fasern im Bereich zwischen 5 bis 10 dtex liegt.

7. Filtereinheit nach einem der Ansprüche 1 bis 6, wobei die Titration der zweiten Fasern im Bereich zwischen 0,5 und 5 dtex liegt.

8. Filtereinheit nach einem der Ansprüche 2 bis 7, wobei die Titration der dritten Fasern im Bereich zwischen 0,5 bis 5 dtex liegt.

9. Filtereinheit nach Anspruch 1 bis 8, wobei die Länge der diskontinuierlichen Fasern im Bereich zwischen 25 mm bis 120 mm liegt.

10. Filtereinheit nach einem der Ansprüche 2 bis 9, wobei die Länge der ersten Fasern verschieden von derjenigen der dritten Fasern ist.

11. Filtereinheit nach einem beliebigen der Ansprüche 1 bis 10, wobei das Tuch (9) des Vorfilters eine Luftdurchlässigkeit aufweist, die im Bereich zwischen 3000 und 12 000 L/m²/s liegt.

12. Filtereinheit nach einem der Ansprüche 1 bis 11, wobei das Tuch (9) des Vorfilters eine Flächenmasse aufweist, die im Bereich zwischen 40 und 90 g/m² liegt.

13. Filtereinheit nach Anspruch 12, wobei das Tuch (9) des Vorfilters eine Oberflächenmasse aufweist, die im Bereich zwischen 50 und 80 g/m² liegt.

14. Filtereinheit nach einem der Ansprüche 1 bis 13, wobei das Tuch (9) des Vorfilters ein wärmeverfestigtes kardiertes Tuch ist, um ihn kohärent zu machen.

15. Filtereinheit nach einem der Ansprüche 1 bis 14, wobei die Fasern des Tuchs (9) des Vorfilters (9) durch einen Verbindungsprozess durch Luftdurchströmung verbunden sind.

16. Filtereinheit nach einem der Ansprüche 1 bis 15, wobei das Medium zum Entfernen von Leukozyten (8) eine oder mehrere Schichten (10) eines Vliesmaterials aus aufgeblasenen Fasern in geschmolzenem Zustand umfasst.

17. Filtereinheit nach Anspruch 16, wobei die Fasern des Mediums zum Entfernen von Leukozyten (8) ausgewählt sind aus den Fasern aus Polyethylen, Polypropylen, Polyethylenterephtalat, Polybutylenterephtalat und ihren Copolymeren.

18. Filtereinheit nach Anspruch 16 oder 17, wobei jede Schicht (10) des Mittels zum Entfernen von Leukozyten eine Luftdurchlässigkeit aufweist, die geringer als diejenige des Tuchs (9) des Vorfilters ist.

19. Filtereinheit nach Anspruch 18, wobei jede Schicht (10) des Mittels zum Entfernen von Leukozyten eine Durchlässigkeit aufweist, die im Bereich zwischen 90 und 500 L/m²/s liegt.

20. Filtereinheit nach einem beliebigen der Ansprüche 1 bis 19, wobei der Vorfilter (7) außerdem nachgelagert vom Tuch (9) des Vorfilters eine oder mehrere Schichten (11) eines porösen Vliesmaterials umfasst, ausgewählt aus der Gruppe, umfassend ein Vlies aus aufgeblasenen Fasern in geschmolzenem Zustand oder ein Spinnvlies.

21. System mit Beuteln (12) zum Entfernen von Leukozyten eines Fluids wie z. B. Blut oder einer Blutverbindung, **dadurch gekennzeichnet, dass** es einen Beutel (13) zum Sammeln des Filtrats umfasst, wobei der Beutel (13) mit Hilfe eines Rohrs (14) und auf dem Niveau einer Eintrittsöffnung mit einer Austrittsöffnung einer Filtereinheit (1) nach einem der Ansprüche 1 bis 20 verbunden ist.

## Claims

1. Filtration unit (1) intended to enable the leucocyte depletion of blood or of a blood component, comprising an external shell (2) equipped with at least one inlet orifice (3) and at least one outlet orifice (4), the shell containing a porous element inserted between said orifices, said porous element containing at least one prefilter (7) for removing aggregates and at least one leucocyte depletion medium (8) using leucocyte adsorption and/or filtration, the prefilter (7) for removing aggregates comprising at least one cohesive non-woven nap (9) **characterised in that** the nap (9) comprises a mixture of first discontinuous thermoplastic fibres and of second discontinuous thermoplastic fibres wherein a melting point is less than that of said first fibres, said first and second fibres being connected by thermal junctions to one another.

2. Filtration unit according to claim 1, wherein the non-woven nap (9) of the prefilter comprises third discontinuous thermoplastic fibres.

3. Filtration unit according to claim 2, wherein the first and third fibres are made of the same thermoplastic polymer.

4. Filtration unit according to one of claims 1 to 3, wherein the second thermoplastic fibres are bicomponent fibres made from two separate thermoplastic polymers coextruded to form a single fibre.

5. Filtration unit according to claim 4, wherein the nap (9) of the prefilter comprises between 50 and 80% by mass of bicomponent fibres.

6. Filtration unit according to one of claims 1 to 5, wherein the count of the first fibres is within the range from 5 to 10 dtex.

7. Filtration unit according to one of claims 1 to 6, wherein the count of the second fibres is within the range from 0.5 to 5 dtex.

8. Filtration unit according to one of claims 2 to 7, wherein the count of the third fibres is within the range from 0.5 to 5 dtex.

9. Filtration unit according to claim 1 to 8, wherein the length of the discontinuous fibres is within the range from 25 mm to 120 mm.

10. Filtration unit according to one of claims 2 to 9, wherein the length of the first fibres is different to that of the third fibres.

11. Filtration unit according to any one of claims 1 to 10, wherein the nap (9) of the prefilter has a permeability to air within the range from 3000 to 12,000 L/m²/s.

12. Filtration unit according to one of claims 1 to 11, wherein the nap (9) of the prefilter has a basis weight within the range from 40 to 90 g/m².

13. Filtration unit according to claim 12, wherein the nap (9) of the prefilter has a basis weight within the range from 50 to 80 g/m².

14. Filtration unit according to one of claims 1 to 13, wherein the nap (9) of the prefilter is a carded nap thermobonded to render same cohesive.

15. Filtration unit according to one of claims 1 to 14, wherein the fibres of the nap (9) of the prefilter are bonded by a through-air bonding method.

16. Filtration unit according to one of claims 1 to 15, wherein the leucocyte depletion medium (8) comprises one or a plurality of layers (10) of a non-woven material of melt-blown fibres.

17. Filtration unit according to claim 16, wherein the fibres of the leucocyte depletion medium (8) are chosen from polyethylene, polypropylene, polyethylene terephthalate, polybutylene terephthalate and copolymers thereof.

18. Filtration unit according to claims 16 or 17, wherein each layer (10) of the leucocyte depletion medium has a permeability to air less than that of the nap (9) of the prefilter.

19. Filtration unit according to claim 18, wherein each layer (10) of the leucocyte depletion medium has a permeability within the range from 90 to 500 L/m²/s.

20. Filtration unit according to any one of claims 1 to 19, wherein the prefilter (7) further comprises downstream from the nap (9) of the prefilter, one or a plurality of layers (11) of a porous non-woven material chosen in the group comprising a non-woven web of melt-blown fibres or a bonded spun non-woven web.

21. System of bags (12) for the leucocyte depletion of a fluid such as blood or a blood component, **characterised in that** it comprises a bag (13) for collecting filtrate, said bag (13) being connected, by means of a tube (14) and at an inlet orifice, to an outlet orifice of a filtration unit (1) according to one of claims 1 to 20.
